# FASCICULE DE BREVET EUROPEEN

(11) **EP 1 403 275 B1**
(45) Date de publication et mention de la délivrance du brevet: **19.10.2005**
(21) Numéro de dépôt: 03290485.6
(22) Date de dépôt: 28.02.2003
(51) Int. Cl.: C07K 5/02, C07D 209/42

(54) **Nouveau procédé de synthèse du perindopril et de ses sels pharmaceutiquement acceptables**
Verfahren für die Synthese von Perindopril und seiner pharmazeutisch annehmbaren Salze
Process for the synthesis of perindopril and its pharmaceutically acceptable salts

(43) Date de publication de la demande: 31.03.2004
(73) Titulaire: Les Laboratoires Servier, 92415 Courbevoie Cedex (FR)
(72) Inventeur: Dubuffet, Thierry, 76210 Bolbec (FR); Langlois, Pascal, 76210 Saint Jean de la Neuville (FR)

(56) Documents cités:
- WO-A-01/58868
- WO-A-03/016336
- VINCENT M ET AL: "Stereoselective Synthesis of a New Perhydroindole Derivative of Chiral Iminodiacid, a Potent Inhibitor of Agiotensin Converting Enzyme" TETRAHEDRON, ELSEVIER SCIENCE PUBLISHERS, AMSTERDAM, NL, vol. 23, no. 16, 1982, pages 1677-1680, XP002155080 ISSN: 0040-4020

## Description

La présente invention concerne un procédé de synthèse du perindopril de formule (I) : 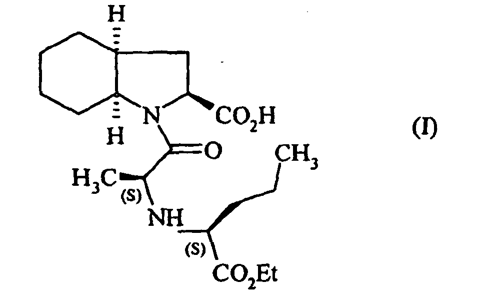 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse performant, facilement transposable à l'échelle industrielle, conduisant au perindopril avec un bon rendement et une excellente pureté, à partir de matières premières bon marché.
Le brevet EP 0 308 341 décrit la synthèse industrielle du perindopril par couplage de l'ester benzylique de l'acide (2*S*, 3a*S*, 7a*S*)-octahydroindole 2-carboxylique avec l'ester éthylique de la N-[(S)-1-carboxybutyl]-(S)-alanine, suivie de la déprotection du groupement carboxylique de l'hétérocycle par hydrogénation catalytique.
Or, l'ester de l'acide (2S, 3aS, 7aS)-octahydroindole-2-carboxylique n'est pas commercial, et sa préparation nécessite plusieurs étapes de synthèse (dont une étape de résolution) à partir de l'acide indole-2-carboxylique.

La demanderesse a présentement mis au point un nouveau procédé de synthèse du perindopril à partir de matières premières aisément accessibles.

Plus spécifiquement, la présente invention concerne un procédé de synthèse du perindopril, et de ses sels pharmaceutiquement acceptables, caractérisé en ce que l'on met en réaction la 1-(1-cyclohexén-1-yl)-pyrrolidine de formule (II) : 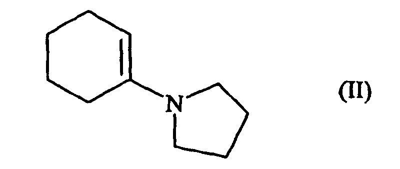 avec le composé de formule (III) : 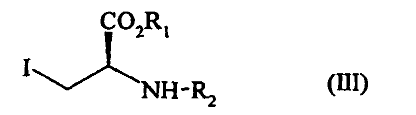 dans laquelle R₁ représente un groupement protecteur de la fonction acide et R₂ représente un groupement protecteur de la fonction amine,
pour conduire au composé de formule (IV) : 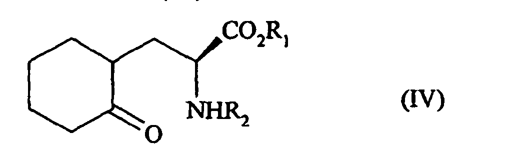 dans laquelle R₁ et R₂ sont tels que définis précédemment,
dont on déprotège la fonction amine avant d'en effectuer la cyclisation suivie de la déshydratation, pour conduire au composé de formule (V) : 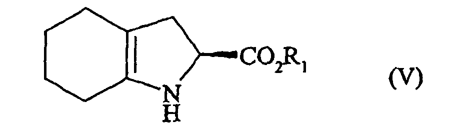 dans laquelle R₁ est tel que défini précédemment,
ou son sel d'addition à un acide minéral ou organique,
que l'on met en réaction avec le composé de formule (VI) : 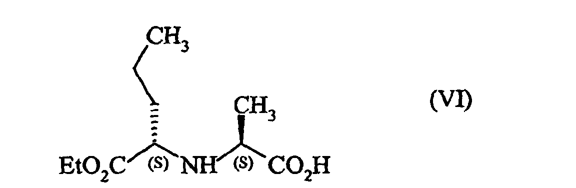 dans l'acétate d'éthyle,
en présence d'une quantité de 1-hydroxybenzotriazole comprise entre 0,4 et 0,6 mole par mole de composé de formule (V) utilisé et d'une quantité de dicyclohexylcarbodiimide comprise entre 1 et 1,2 mole par mole de composé de formule (V) utilisé,
en présence d'une quantité de triéthylamine comprise entre 0,25 et 1,2 mole par mole de composé de formule (V) utilisé,
à une température comprise entre 20 et 77°C,
pour conduire après isolement au composé de formule (VII) : 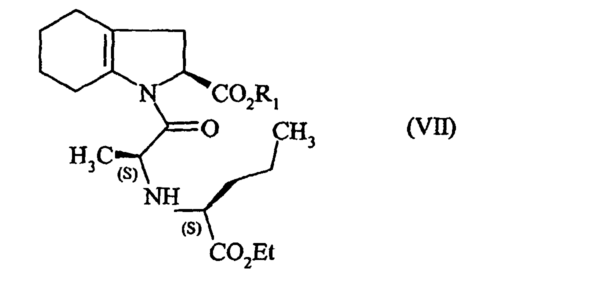 dans laquelle R₁ est tel que défini précédemment,
que l'on hydrogène en présence d'un catalyseur tel que, par exemple, le palladium, le platine, le rhodium ou le nickel,
sous une pression d'hydrogène comprise entre 1 et 30 bars, de préférence entre 1 et 10 bars, pour conduire, après déprotection de la fonction acide, au perindopril de formule (I), que l'on transforme, si on le souhaite, en un sel pharmaceutiquement acceptable tel que le sel de tert-butylamine.

L'exemple ci-dessous illustre l'invention, mais ne la limite en aucune façon.

### EXEMPLE : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S)-2-[(Tert-butyloxycarbonyl)-amino]-3-(2-oxocyclohexyl)-propanoate de benzyle

Dans un réacteur équipé d'une colonne à reflux, placer 200 g de 1-(1-cyclohexén-1-yl)-pyrrolidine, 535 g de (2S)-2-[(tert-butyloxycarbonyl)-amino]-3-iodopropanoate de benzyle et 1,5 1 d'acétonitrile.
Amener au reflux pendant 1 h, puis ramener le mélange à température ambiante. Après évaporation du solvant, ajouter 2 1 d'eau, puis de acide acétique. Extraire par l'acétate d'éthyle et évaporer à sec. Le (2S)-2-[(tert-butyloxycarbonyl)-amino]-3-(2-oxocyclohexyl)-propanoate de benzyle est ainsi obtenu avec un rendement de 80 %.

### Stade B : (2S)-2-Amino-3-(2-oxocyclohexyl)-propanoate de benzyle

Dans un réacteur, placer 200 g du composé obtenu dans le stade précédent, 1,5 1 de dichlorométhane et 60 g d'acide trifluoroacétique. Après 1 h30 d'agitation à température ambiante, ajouter 2 l d'une solution saturée d'hydrogénocarbonate de sodium. Extraire par le dichlorométhane et évaporer à sec.
Le (2S)-2-amino-3-(2-oxocyclohexyl)-propanoate de benzyle est ainsi obtenu avec un rendement de 90 %.

### Stade C : (2S)-2,3,4,5,6,7-Hexahydro-1H-indole-2-carboxylate de benzyle, paratoluènesulfonate

Dans un réacteur, chauffer à reflux 200 g du composé obtenu dans le stade précédent, 151,9 g d'acide p-toluènesulfonique et 1 l de toluène en éliminant l'eau formée par entraînement azéotropique. Lorsqu'il ne décante plus d'eau, évaporer le toluène.
Le paratoluènesulfonate du (2S)-2,3,4,5,6,7-hexahydro-1*H*-indole-2-carboxylate de benzyle est ainsi obtenu avec un rendement sur brut de 97 %.

### Stade D : (2S)-1-{(2S)-2-[(1S)-1-(Ethoxycarbonyl)-butylamino]-propionyl}-2,3,4,5,6,7-hexahydro-1H-indole-2-carboxylate de benzyle

Dans un réacteur, placer 200 g du composé obtenu dans le stade précédent, 46 g de triéthylamine, 1 l d'acétate d'éthyle puis, après 10 mn d'agitation à température ambiante, 104 g de N-[(S)-carbéthoxy-1-butyl]-(S)-alanine, 30 g de 1-hydroxybenzotriazole et 100 g de dicyclohexylcarbodiimide. Le mélange hétérogène est ensuite porté à 30°C pendant 3 h sous bonne agitation, puis il est refroidi à 0°C et filtré. Le filtrat est ensuite lavé, puis évaporé à sec pour conduire au produit attendu avec un rendement de 95 %.

### Stade E : Acide (2S, 3aS, 7aS)-1-{(2S)-2-[(1S)-1-(éthoxycarbonyl)-butylamino]-propionyl}-octahydro-1H-indole-2-carboxylique

Dans un hydrogénateur, placer 200 g du composé obtenu dans le stade précédent, en solution dans l'acide acétique, puis 5 g de Pt/C à 10 %. Hydrogéner sous pression de 5 bars à température ambiante, jusqu'à absorption de la quantité théorique d'hydrogène.
Eliminer le catalyseur par filtration, puis refroidir entre 0 et 5°C et récolter le solide obtenu par filtration. Laver le gâteau et le sécher jusqu'à poids constant.
L'acide (2S, 3aS, 7aS)-1-{(2S)-2-[(1S)-1-(éthoxycarbonyl)-butylamino]-propionyl}-octahydro-1*H*-indole-2-carboxylique est ainsi obtenu avec un rendement de 85 % et une pureté énantiomérique de 99 %.

### Stade F : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

Le composé obtenu dans le stade précédent (200 g) est mis en solution dans 2,8 l d'acétate d'éthyle, puis 40 g de tert-butylamine et 0,4 1 d'acétate d'éthyle sont ajoutés. La suspension obtenue est ensuite portée au reflux jusqu'à dissolution totale, puis la solution obtenue est filtrée à chaud et refroidie sous agitation jusqu'à une température de 15-20°C.
Le précipité obtenu est alors filtré, réempâté à l'acétate d'éthyle, séché puis broyé pour conduire au produit attendu avec un rendement de 95%.

## Revendications

1. Procédé de synthèse du perindopril de formule (I) : 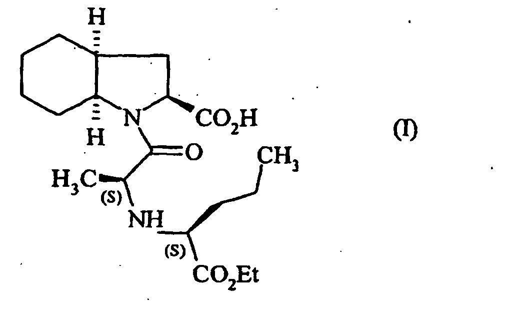 et de ses sels pharmaceutiquement acceptables,
**caractérisé en ce que** l'on met en réaction la 1-(1-cyclohexén-1-yl)-pyrrolidine de formule (II) : 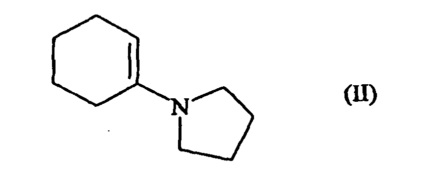 avec le composé de formule (III) : 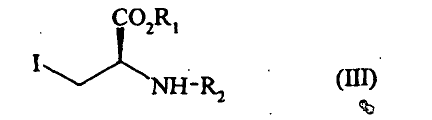 dans laquelle R₁ représente un groupement protecteur de la fonction acide et R₂ représente un groupement protecteur de la fonction amine,
pour conduire au composé de formule (IV) : 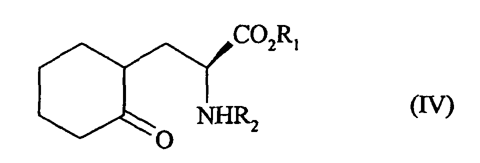 dans laquelle R₁ et R₂ sont tels que définis précédemment,
dont on déprotège la fonction amine avant d'en effectuer la cyclisation suivie de la déshydratation, pour conduire au composé de formule (V) : 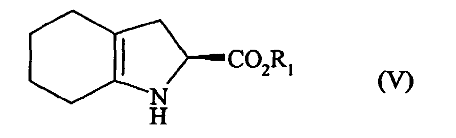 dans laquelle R₁ est tel que défini précédemment,
ou son sel d'addition à un acide minéral ou organique,
que l'on met en réaction avec le composé de formule (VI) : 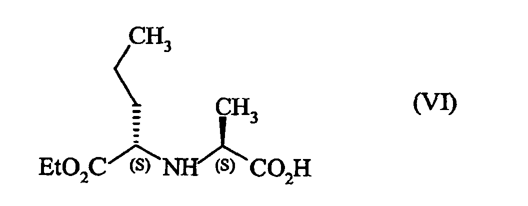 dans l'acétate d'éthyle,
en présence d'une quantité de 1-hydroxybenzotriazole comprise entre 0,4 et 0,6 mole par mole de composé de formule (V) utilisé et d'une quantité de dicyclohexylcarbodiimide comprise entre 1 et 1,2 mole par mole de composé de formule (V) utilisé,
en présence d'une quantité de triéthylamine comprise entre 0,25 et 1,2 mole par mole de composé de formule (V) utilisé,
à une température comprise entre 20 et 77°C,
pour conduire après isolement au composé de formule (VII) : 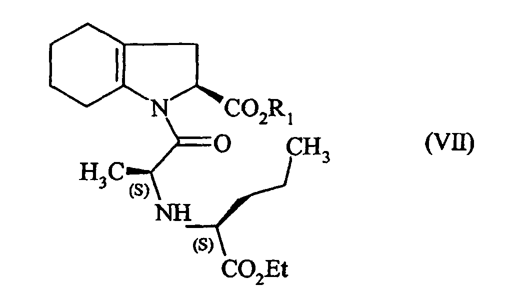 dans laquelle R₁ est tel que défini précédemment,
que l'on hydrogène en présence d'un catalyseur tel que le palladium, le platine, le rhodium ou le nickel,
sous une pression d'hydrogène comprise entre 1 et 30 bars, pour conduire, après déprotection de la fonction acide, au perindopril de formule (I), que l'on transforme, si on le souhaite, en un sel pharmaceutiquement acceptable tel que le sel de tert-butylamine.

2. Procédé de synthèse selon la revendication 1, **caractérisé en ce que** la pression d'hydrogène de la réaction d'hydrogénation est comprise entre 1 et 10 bars.

3. Procédé de synthèse selon la revendication 1 du perindopril sous sa forme de sel de tert-butylamine.

## Patentansprüche

1. Verfahren zur Synthese von Perindopril der Formel (I): 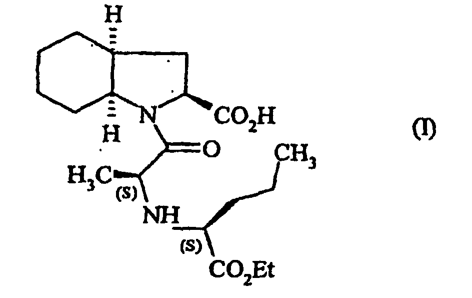 und von dessen pharmazeutisch annehmbaren Salzen,
**dadurch gekennzeichnet, daß** man 1-(1-Cyclohexen-1-yl)-pyrrolidin der Formel (II) : 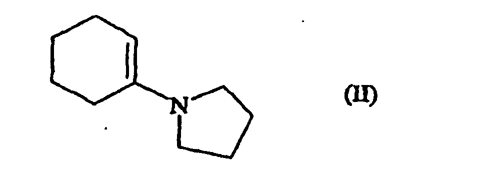 mit der Verbindung der Formel (III): 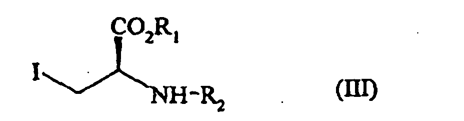 in der R₁ eine Schutzgruppe für die Säurefunktion und R₂ eine Schutzgruppe für die Aminfunktion bedeuten, umsetzt,
zur Bildung der Verbindung der Formel (IV): 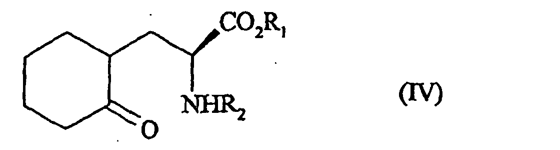 in der R₁ und R₂ die oben angegebenen Bedeutungen besitzen,
von der man die Schutzgruppe der Aminfunktion abspaltet, bevor man die Cyclisierung, gefolgt von der Dehydratisierung, durchführt zur Bildung der Verbindung der Formel (V): 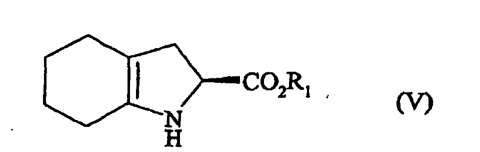 in der R₁ die oben angegebenen Bedeutungen besitzt,
oder des Additionssalzes mit einer anorganischen oder organischen Säure,
welche man in Ethylacetat in Gegenwart einer Menge 1-Hydroxybenzotriazol zwischen 0,4 und 0,6 Mol pro Mol der Verbindung der Formel (V) und einer Menge Dicyclohexylcarbodiimid zwischen 1 und 1,2 Mol pro Mol der verwendeten Verbindung der Formel (V),
in Gegenwart einer Menge Triethylamin zwischen 0,25 und 1,2 Mol pro Mol der verwendeten Verbindung der Formel (V)
bei einer Temperatur zwischen 20 und 77°C mit der Verbindung der Formel (VI): 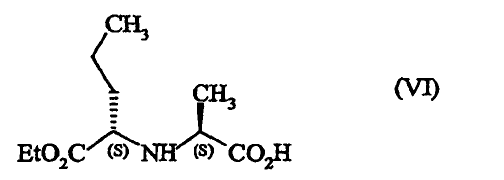 umsetzt,
so daß man nach der Isolierung die Verbindung der Formel (VII) erhält: 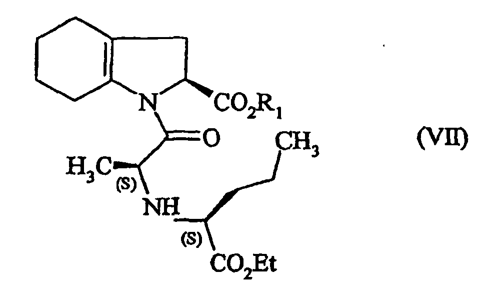 in der R₁ die oben angegebenen Bedeutungen besitzt,
welche man in Gegenwart eines Katalysators, wie Palladium, Platin, Rhodium oder Nickel,
bei einem Wasserstoffdruck zwischen 1 und 30 bar hydriert, so daß man nach der Abspaltung der Schutzgruppe der Säurefunktion Perindopril der Formel (I) erhält, welches man gewünschtenfalls in ein pharmazeutisch annehmbares Salz, wie das tert.-Butylaminsalz, umwandelt.

2. Syntheseverfahren nach Anspruch 1, **dadurch gekennzeichnet, daß** der Wasserstoffdruck bei der Hydrierreaktion zwischen 1 und 10 bar liegt.

3. Verfahren nach Anspruch 1 zur Synthese von Perindopril in Form seines tert. - Butylaminsalzes.

## Claims

1. Process for the synthesis of perindopril of formula (I) : 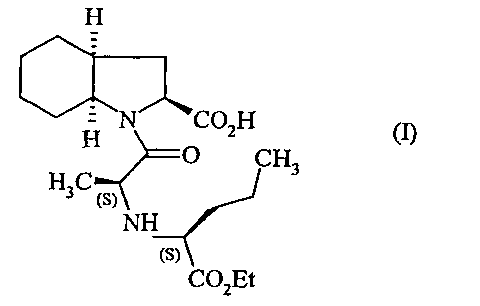 and pharmaceutically acceptable salts thereof,
**characterised in that** 1-(1-cyclohexen-1-yl)-pyrrolidine of formula (II) : 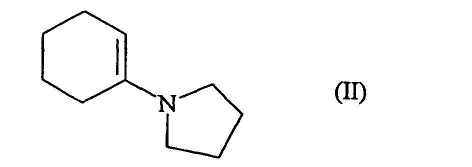 is reacted with the compound of formula (III) : 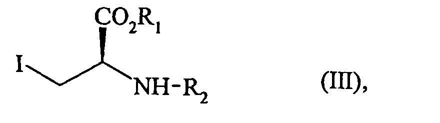 wherein R₁ represents a protecting group for the acid function and R₂ represents a protecting group for the amine function,
to yield the compound of formula (IV) : 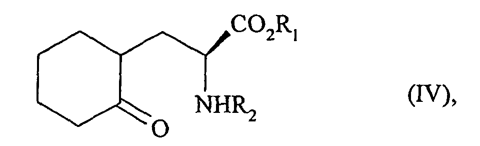 wherein R₁ and R₂ are as defined hereinbefore,
the amine function of which is deprotected before cyclisation is carried out, followed by dehydration, to yield the compound of formula (V) : 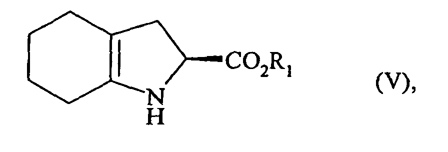 wherein R₁ is as defined hereinbefore,
or an addition salt thereof with a mineral or organic acid,
which is reacted with the compound of formula (VI) : 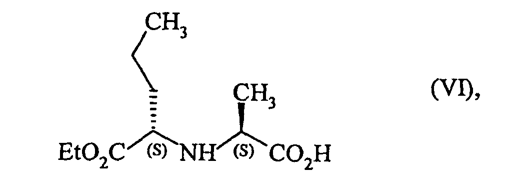 in ethyl acetate,
in the presence of an amount of 1-hydroxybenzotriazole of from 0.4 to 0.6 mol per mol of compound of formula (V) used and an amount of dicyclohexylcarbodiimide of from 1 to 1.2 mol per mol of compound of formula (V) used,
in the presence of an amount of triethylamine of from 0.25 to 1.2 mol per mol of compound of formula (V) used,
at a temperature of from 20 to 77°C,
to yield, after isolation, the compound of formula (VII) : 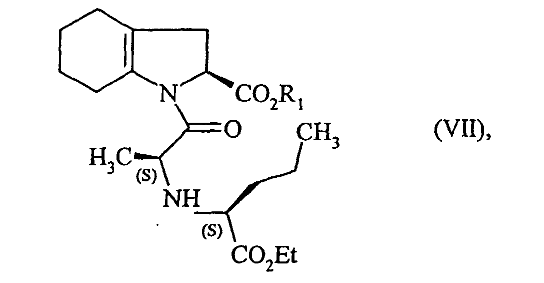 wherein R₁ is as defined hereinbefore,
which is hydrogenated in the presence of a catalyst such as palladium, platinum, rhodium or nickel,
under a hydrogen pressure of from 1 to 30 bars, to yield, after deprotection of the acid function, perindopril of formula (I), which is converted, if desired, into a pharmaceutically acceptable salt such as the tert-butylamine salt.

2. Synthesis process according to claim 1, **characterised in that** the hydrogen pressure in the hydrogenation reaction is from 1 to 10 bars.

3. Process according to claim 1 for the synthesis of perindopril in the form of its tert-butylamine salt.
